(19) Europäisches Patentamt
European Patent Office
Office européen des brevets

(11) **EP 3 053 458 B1**

(12) **EUROPEAN PATENT SPECIFICATION**

(45) Date of publication and mention
of the grant of the patent:
**30.10.2019 Bulletin 2019/44**

(51) Int Cl.:
*A61K 38/39* (2006.01)     *A61K 9/16* (2006.01)
*A61K 31/737* (2006.01)     *A61K 36/736* (2006.01)
*A23L 33/105* (2016.01)     *A23L 33/18* (2016.01)
*A61K 31/375* (2006.01)

(21) Application number: **15152822.1**

(22) Date of filing: **28.01.2015**

(54) **Spray-Dried Composition Comprising an Acerola Fruit Extract, Hydrolyzed Collagen Type II and Chondroitin Sulfate**

Sprühgetrocknete Zusammensetzung, die einen Acerolafruchtextrakt, hydrolysiertes Kollagen vom Typ II und Chondroitinsulfat umfasst

Composition séchée par atomisation comprenant un extrait de fruit d'acérola, du collagène hydrolysé de type II et du sulfate de chondroïtine

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.08.2016 Bulletin 2016/32**

(73) Proprietor: **Paninkret Chemisch-Pharmazeutisches Werk GmbH 25364 Westerhorn (DE)**

(72) Inventor: **Klüwer, Per 25462 Rellingen (DE)**

(74) Representative: **Uexküll & Stolberg Partnerschaft von Patent- und Rechtsanwälten mbB Beselerstraße 4 22607 Hamburg (DE)**

(56) References cited:
EP-A1- 2 724 628     WO-A1-2014/204866
WO-A2-2009/080778     CN-A- 102 349 655
DE-A1- 10 217 808     DE-A1-102009 030 351

US-A1- 2003 091 652

- ROBERTA DA SILVA NUNES ET AL: "Antigenotoxicity and Antioxidant Activity of Acerola Fruit (L.) at Two Stages of Ripeness", PLANT FOODS FOR HUMAN NUTRITION, KLUWER ACADEMIC PUBLISHERS, DO, vol. 66, no. 2, 19 April 2011 (2011-04-19), pages 129-135, XP019926077, ISSN: 1573-9104, DOI: 10.1007/S11130-011-0223-7
- ERIKO UCHIDA ET AL: "Absorption and Excretion of Ascorbic Acid Alone and in Acerola (Malpighia emarginata) Juice: Comparison in Healthy Japanese Subjects", BIOL PHRM. BULL., vol. 34, no. 11, 30 November 2011 (2011-11-30), pages 1744-1747, XP055203503,
- Vesna T Tumbas ET AL: "Effect of rosehip (Rosa canina L.) phytochemicals on stable free radicals and human cancer cells", JOURNAL OF THE SCIENCE OF FOOD AND AGRICULTURE, vol. 92, no. 6, 1 April 2012 (2012-04-01), pages 1273-1281, XP055484750, GB ISSN: 0022-5142, DOI: 10.1002/jsfa.4695
- RIGHETTO A M ET AL: "CHEMICAL COMPOSITION AND ANTIOXIDANT ACTIVITY OF JUICES FROM MATURE AND IMMATURE ACEROLA (MALPIGHIA EMARGINATA DC)", FOOD SCIENCE AND TECHNOLOGY INTERNATI, SAGE PUBLICATIONS, NEW YORK, NY, US, vol. 11, 1 January 2005 (2005-01-01), pages 315-321, XP009072874, ISSN: 1082-0132, DOI: 10.1177/1082013205056785

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**SUMMARY OF THE INVENTION**

The present invention relates to a method according to claims 1-4 for preparing a spray-dried composition comprising an Acerola fruit extract and hydrolyzed collagen, the method comprising the steps of mixing a liquid Acerola fruit extract obtained by pressing of an Acerola fruit and a fluid comprising hydrolyzed collagen type II and at least 5 % by weight of the fluid chondroitin sulfate and spray-drying the mixture. Further, the invention also relates to a spray-dried composition according to claims 5-8 prepared by this method, such as a composition comprising (a) hydrolyzed collagen type II and chondroitin sulfate; and (b) Acerola fruit extract comprising vitamin C and all different compounds that are comprised in Acerola fruit juice; wherein the composition comprises preferably no spray-drying carrier other than hydrolyzed collagen, in particular no maltodextrine, oxidized starch or lactose.

**TECHNICAL BACKGROUND**

Physical exercise, older age or over-weight are often associated with a deteriorating joint health. In particular, cartilaginous joints are in danger of deterioration when strained beyond their capacity. Loss of the cartilage in the joints can damage the joints and lead to secondary diseases such as arthritis. Therefore, there is a need for food supplements that support cartilage formation and/or metabolism.

It has been shown in the art that intake of hydrolyzed collagen type II helps to protect the joints and reduces the risk of joint problems (Benito-Ruiz et al., 2009, Int J Food Sci Nutr, 60(S2):99-113). Further, a study by Clark et al. showed that collagen hydrolysate improves joint health in athletes and pain due to high athletic activity may be reduced (Cur Med Res Op, 2008, 24(5), 1485-1496).

Collagen type II is one of the fiber-forming collagens and mainly found in cartilage. Collagens contribute about 60% of the dry weight of cartilage. Collagen type II is composed of strong fibrils, stabilized by disulfide bridges, and made up of convoluted polypeptide chains. The unique structure of collagen type II provides an elastic joint compound.

Dietary supplementation with collagen type II for joint cartilage health has been proposed in the art because collagen is able to provide the unique amino acids hydroxyproline and hydroxylysine that are not found in other animal or human proteins.

However, the absorption of orally ingested, full-length collagen is poor. This drawback can be avoided by hydrolyzation of collagen, which results in well-absorbable hydrolyzed collagen. Collagen hydrolysate is a nutritional supplement that has been shown to exert an anabolic effect on cartilage tissue and appears to be beneficial in patients with osteoarthritis. It has been pre-clinically demonstrated that collagen hydrolysate passes across the mucosal barrier in the small intestine, accumulates in cartilage tissue, and stimulates the production of Type II collagen, which is the major protein in articular collagen, and proteoglycans in the extracellular matrix of cartilage (Oesser et al., 1999, J Nutr, 1891-5; Oesser et al., 2003, Cell Tissue Res, 311: 393-9; Oesser et al., 2005, Orthopaedische Praxis, 565-8). Additionally, several clinical studies provided evidence of a beneficial effect of the administration of collagen hydrolysate on joint health in a variety of patient populations, such as patients having osteoarthritis or exercise-related joint pain (e.g. Moskowitz, 2000, Semin Arthritis Rheum, 30: 87-99; Zuckley et al., 2004, Med Sci Sports Exerc, 37(Suppl):S 153-S4).

Usually, collagen hydrolysate is made out of collagenous tissue from porcine sources such as bone, hide and hide split by subjecting these sources to enzymatic hydrolysis and subsequent purification.

A key role in the biosynthesis and maintenance of collagenous tissues as well as in the formation of bones, teeth, muscles and skin is played by vitamin C which is essential for the biosynthesis of collagen (Naidu, 2003, Nutr J, 7-16). It would therefore be advantageous to provide hydrolyzed collagen in combination with vitamin C. Vitamin C is a co-factor for a number of enzymes which require transition metal ions, such as for the prolyl hydroxylase which is essential for the collagen synthesis in mammals. In the relevant reaction, vitamin C is utilized as a co-factor to reduce the iron ion $Fe^{3+}$ to the ferrous state ($Fe^{2+}$), thus keeping the enzyme prolyl hydroxylase active. Vitamin C is therefore essential for the maintenance of healthy connective tissue. Moreover, vitamin C has several other important functions that make it particularly suitable as an additive for a food supplement. For example, vitamin C acts as an antioxidant in the preservation of e.g. dry and frozen fruits and feed, namely in juices, or after consumption in the mammalian body where vitamin C acts as a protector against oxidative stress. The vitamin is also imperative for the immune system and if the body is not adequately supplied with vitamin C, receding gums, tooth loss, vascular weakness and bleeding (all associated with a disturbed collagen synthesis due to vitamin C-shortage) are the consequences. Not least, vitamin C also increases the iron absorption.

As the use of synthetic food supplements has lately fallen into disrepute among consumers, it is desirable to provide hydrolyzed collagen in combination with natural vitamin C. In particular, synthetic vitamin C, which consists mainly of isolated ascorbic acid, is thought to be not as effective as natural vitamin C. Natural vitamin C is not only thought to have a higher bioavailability but is also accompanied by further positive substances, such as secondary plant compounds. In addition, natural vitamin C can be added during product manufacture without losing the right to label a product as "clean label" product. One way of providing high levels of natural vitamin C is the use of extracts from the Acerola cherry, which is particularly rich in vitamin C. While Acerola is already frequently used in South America and Japan, the USA and Europe are still great potential markets to be explored.

As vitamin C quickly deteriorates, in particular when in solution, it is advantageous to provide vitamin C-containing food supplements in dried form, e.g. in a spray-dried form. Also other nutritive substances comprised in Acerola are preserved by drying into a powder. Furthermore, a dried form allows the precise dosing of a sufficient amount of vitamin C and hydrolyzed collagen. The dried food supplement is, further, better suited for the manufacture of a number of different food products (e.g. nutrition bars) than a solution comprising vitamin C and collagen. Another advantage of the dried powder form is the reduced transport weight and volume.

WO2009/080778 discloses the use of hydrolyzed collagen as a protective drying agent in the preparation of a spray dried plant extract powder, such as rosehip extract, thyme extract and saliva extract.

US2003/091652 discloses powders comprising spray-dried collagen type-II hydrolysate comprising chondroitin sulfate which is blended with vitamin C.

However, traditional drying methods, such as spray drying or vacuum band drying have different drawbacks. For example, a spray-dried Acerola fruit extract treated in accordance with traditional spray-drying methods would comprise spray-drying carriers, such as maltodextrin, which reduce the content of natural vitamin C in the final food supplement considerably and have very low nutritional value. Such carriers also often negatively affect the taste of dried extracts and are thought to potentially have negative side effects. When an Acerola fruit extract is dried by other means, for example by vacuum belt drying, the resulting granulate is less fine than after spray-drying. After vacuum belt drying, the powder would therefore have to be ground to achieve a comparable degree of fineness. However, grinding creates heat and increases the hygroscopy which increases clumping of the powder. In addition, spray-drying is better suited to provide a product compliant with precise quality standards regarding particle size distribution, residual moisture content, bulk density and morphology (Patel et al., 2009, Ind J Sci Techn, 2(10):44-47), as well as good flowability, good solubility, and low hygroscopy.

In summary, it can be concluded that there is a need in the art for stable powder compositions which comprise hydrolyzed collagen and high levels of natural vitamin C with good or neutral flavor.

## DESCRIPTION OF THE INVENTION

In the context of the present invention, it has surprisingly been found that collagen hydrolyzate can serve as a spray-drying carrier for Acerola fruit extract without the addition of regular, nutritionally ineffective spray-drying carriers, such as maltodextrine, lactose or oxidized starch.

The invention thereby provides a combination of highly bioavailable collagen and natural vitamin C as a cofactor as set forth in the appended claims while containing only valuable and desired natural components. The spray-dried composition according to the present invention as set forth in claim 5 is more compact than prior art products and can be processed without additives in so-called "clean label" products. Moreover, the concentration of natural vitamin C in the composition is particularly high, such as about 4% by weight, while prior art products contain usually less vitamin C and/or a high level of synthetic vitamin C.

Thus, the present invention provides a method for preparing a spray-dried composition comprising an Acerola fruit extract and hydrolyzed collagen, the method comprising the steps of (a) mixing a liquid Acerola fruit extract obtained by pressing of an Acerola fruit and a fluid comprising hydrolyzed collagen type II and at least 5 % by weight of the fluid chondroitin sulfate; and (b) spray-drying the mixture. The hydrolyzed collagen is hydrolyzed collagen type II, namely hydrolyzed collagen type II comprised in cartilage-derived material. The liquid Acerola fruit extract comprises vitamin C and all different compounds that are comprised in Acerola fruit juice.

In a second aspect, the present invention relates to a spray-dried composition according to claim 5 obtainable according to the method, comprising (a) cartilage-derived material which comprises the hydrolyzed collagen type II and chondroitin sulfate; and (b) Acerola fruit extract comprising vitamin C and all different compounds that are comprised in Acerola fruit juice. Such a spray-dried composition will preferably comprise no spray-drying carrier other than compounds comprised in the cartilage-derived material or (potentially) in the Acerola fruit extract. In particular, the present invention relates to a spray-dried composition as described above; wherein the composition comprises no maltodextrine, lactose, or oxidized starch. Preferably, the spray-dried composition according to the present invention consists of an Acerola fruit extract (comprising vitamin C and all different compounds that are comprised in Acerola fruit juice) and cartilage-derived material which comprises the hydrolyzed collagen type II and chondroitin sulfate. In this embodiment, the compositions do not comprise additional compounds or ingredients, thereby providing a maximum concentration of the beneficial compounds comprised in the Acerola fruit extract and the cartilage-derived material.

A spray-dried composition in the sense of the present invention is a composition that has been dried by a spray drying technique. During spray drying, a liquid input stream is sprayed through a nozzle (atomized) into a stream of hot air and vaporized. Due to the heat, the moisture comprised in the vapor droplets quickly evaporates leaving the solid spray-dried powder behind. Spray-drying techniques and apparatuses are well known in the art (compare e.g. Patel et al., 2009, Ind J Sci Techn, 2(10):44-47) and described in detail elsewhere herein.

As a result of the spray drying process, the spray-dried composition of the invention is a powder, in particular a dry powder. The spray-dried composition has a low moisture content. The spray-dried composition will usually have a moisture content of below about 6% by weight, usually below about 5% by weight, and preferably below about 4% by weight. In a particularly preferred embodiment, the moisture content is below about 3% by weight. In other words, the spray-dried composition has a moisture content of between 0% and 6% by weight, preferably between 0% and 5% by weight, more preferably between 0% and 4% by weight. In a particularly preferred embodiment, the spray-dried composition has a moisture content that is between 0% and 3%.

The dry powder form of the composition according to claim 5 consists of particles comprising all compounds from the Acerola fruit juice, chondroitin sulfate and cartilage-derived material comprising hydrolyzed collagen type II.

The spray drying process is carried out under conditions that result in a substantially amorphous finely divided dry powder consisting essentially of particles within the desired size range noted below. Consisting "essentially" in this context means that at least 85%, preferably at least 90%, more preferably at least 95% of all particles in the spray dried composition are within the indicated size range. Preferably, the spray dried composition consists essentially of particles having a size of less than about 300 $\mu$m mean diameter, more preferably less than about 250 $\mu$m, even more preferably less than about 200 $\mu$m. In a particularly preferred embodiment, the composition consists essentially of particles having a size in the range of about 50 $\mu$m to about 250 $\mu$m mean diameter, most preferred in the range of about 100 $\mu$m to about 200 $\mu$m mean diameter.

Due to the spray drying process, the powder is sufficiently fine, so that no additional grinding step is required. Thus, preferably, no grinding step is carried out after step b) during the method of the invention. In consequence, the powder is not exposed to the humidity in the air in an additional grinding step, thus, reducing the risk of agglutination/clumping. Thus, the spray-dried compositions of the invention comprise substantially no or very few clumps. This means that less than 20%, 15%, 10%, 5%, 4%, 3%, 2%, or 1% by weight of the dry matter in the spray-dried compositions is clumped (when stored sealed, e.g. under vacuum, e.g. after storage for 1 week, 1 month or 1 year). Preferably less than 5% by weight of the dry matter in the spray-dried compositions is clumped. More preferably, less than 1% by weight of the dry matter is clumped in the spray-dried compositions.

Acerola is a tropical fruit-bearing shrub that belongs to the genus *Malpighia* and is presently known under the scientific name *Malpighia emarginata* D.C., even though other names such as *Malpighia glabra* L. and *Malpighia punicifolia* have been used synonymously for the plant in the past. The Acerola fruit extract according to the present invention can be obtained from different species/varieties of the genus *Malpighia.* However, it is particularly preferred that the extract is manufactured starting from plants of the species *Malpighia emarginata (Malpighia glabra L., Malpighia punicifolia).*

The extract is obtained by disruption of cells from a fruit of a plant of the species *Malpighia emarginata* by pressing of the Acerola fruit. The Acerola fruit is drupaceous with a round to conic form and red, purple or yellow color when ripe. Methods for the preparation of Acerola fruit extracts are well known in the art. In one embodiment of the disclosure, material of an Acerola fruit is provided. Suitable starting materials are Acerola fruits in any developmental fruit ripening stage. The Acerola fruit in the sense of the present invention can be immature to ripe mature. It is known, that the content of vitamin C reduces in the Acerola fruit during the ripening process. For example, while immature Acerola fruit have an ascorbic acid content of e.g. about 2400 mg*100 g-1 fresh pulp, the content in ripe mature fruit is e.g. about 950 mg*100 g-1 fresh pulp (Assis et al., 2008, Fruits, 63(2):93-101). On the other hand, it is known that the ripe mature fruit have a better taste and a more appealing color. Therefore, it is preferred that the Acerola fruit extract is derived from a mixture of Acerola fruits comprising immature as well as ripe mature fruits. This way, a spray-dried product can be achieved that has a good taste, a high content of natural vitamin C and an appealing color. It will be understood that the vitamin C comprised in the Acerola extract is from Acerola fruit, i.e. derived from Acerola from. This means that the vitamin C comprised in the Acerola extract is natural vitamin C as defined elsewhere herein.

Apart from the high vitamin C content, Acerola is also rich in pectin and pectolytic enzymes, antioxidants such as carotinoids, plant fibre, vitamins, such as vitamin B, and several biofunctional substances, thiamin, riboflavin, niacin, proteins and mineral salts (Assis et al., 2008, Fruits, 63(2):93-101). As a result of the production procedure described elsewhere herein, the Acerola fruit extract according to the present invention comprises a mixture of different compounds that were originally comprised in the Acerola fruit. These compounds are designated "compounds from the Acerola fruit extract" herein. Accordingly, the (liquid) Acerola fruit extract also comprises one or several compounds selected from the group consisting of vitamins C, A, B1, B2, B3, carotenoids and bioflavonoids.

In consequence, as used herein, the term "Acerola fruit extract" describes a mixture of different substances/compounds from the Acerola fruit (i.e. substances/compounds that are comprised in Acerola fruit). A composition consisting only of isolated vitamin C does not fall under the term "Acerola fruit extract" as used herein as will be understood by the person skilled in the art. This means that the Acerola fruit extract comprises vitamin C and all different compounds that are comprised in Acerola fruit juice. Other compounds from the Acerola fruit that are vitamins include, but are not limited to thiamine (vitamin B1), riboflavin (vitamin B2), niacin (vitamin B3), pantothenic acid (B5), vitamin B6, folate (B9), and the like. It is nevertheless preferred that the Acerola fruit extract is a whole fruit extract. A whole fruit extract comprises essentially all compounds that are comprised in Acerola fruit pulp. Thus, more specifically, it is preferred that the Acerola fruit extract is a whole fruit pulp extract.

This means that the Acerola fruit extract comprised in the spray dried compositions of the invention comprises essentially all different compounds that are comprised in Acerola fruit juice. "Different compounds" refers to compounds that differ in their chemical structure, i.e. to different types of compounds. While the extract is deprived of water molecules during the spray drying procedure, further compounds that are comprised in the fruit juice are preserved during the spray drying procedure and form the spray dried composition (powder) together with the hydrolyzed collagen. Thus, the Acerola extract comprised in the spray dried composition comprises 100% of the different compounds that are comprised in Acerola fruit juice.

The Acerola fruit is a drupe comprising the *pericarp* and seeds. The *pericarp* of fruit is generally subdivided into the layers *epicarp, mesocarp* and *endocarp.* The *epicarp* comprises the fruit skin. The pulp (flesh) of the fruit is surrounding the seed. The Acerola fruit extract contains substantially no seed-derived material, i.e. the seeds are at least partially removed during the processing of the fruit. The Acerola fruit extract is, thus, preferably an Acerola fruit pulp extract. The Acerola fruit pulp extract may or may not comprise compounds derived from the fruit skin.

The Acerola fruit material will usually initially be pressed/squeezed, crushed or chopped, which leads to disruption of the plant cell wall. Thus, the liquid Acerola fruit extract for use in the method of the invention can e.g. be an Acerola juice, such as an Acerola pressed juice/squeezed juice. In a preferred embodiment, the liquid Acerola fruit extract is an Acerola juice. In a more preferred embodiment, the liquid Acerola fruit extract is an Acerola pressed juice. A pressed juice is prepared e.g. by mechanically squeezing or macerating the fruit, and optionally filtering the juice. The pressed juice is then directly bottled or otherwise stored for future use. However, the liquid Acerola fruit extract can also be produced from Acerola pomace. Ways of producing an Acerola fruit extract for use in the method of the invention (e.g. a pressed Acerola juice) are well known in the art.

The Acerola fruit extract for use in the method of the present invention is in liquid form, i.e. is a liquid Acerola fruit extract. While the different terms "liquid" and "fluid" are used herein to discriminate between liquid/fluid compositions that comprise Acerola and hydrolyzed collagen, respectively, this shall not imply that the terms "liquid" and "fluid" have a different meaning. In fact, "liquid" and "fluid" have the same meaning and could be used interchangeably.

In particular, the liquid Acerola fruit extract used in the method of the invention is not in solid form, such as in form of a powder. Preferably, the liquid Acerola fruit extract has a water content of above about 10% by weight, usually above about 20% by weight, and preferably above about 30% by weight. In other words, the liquid Acerola fruit extract has a water content of from 10% to 95%, from 20% to 80%, preferably from 30% to 70%, more preferably from 40% to 60% (by weight). In one embodiment, the liquid Acerola fruit extract has a water content of from 20% to 80% by weight. In a preferred embodiment, the liquid Acerola fruit extract has a water content of from 40% to 60% by weight. In a particularly preferred embodiment, the liquid Acerola fruit extract has a water content of about 50% by weight. Suitable liquid Acerola fruit extracts can be purchased from different manufacturers (e.g. NutriBotanica (Brazil), Niagro - Nichirei do Brasil Agrícola Ltda. (Brazil), Fruteza Sucos Naturais Ltda. (Brazil)).

The term "liquid extract" comprises concentrated and non-concentrated liquid Acerola extracts. Nevertheless, it is preferred that the liquid Acerola fruit extract is concentrated after the above described extracting procedure. Methods for the concentration of fruit extracts are known in the art. During the concentration, water is removed from the extract and the advantageous compounds comprised in Acerola can be provided in an even higher concentration. Thus, in this embodiment, the liquid Acerola fruit extract is a liquid Acerola fruit extract concentrate. For example, the liquid Acerola fruit extract may be an Acerola juice concentrate. All properties of the liquid Acerola fruit extract described herein pertain also to the liquid Acerola fruit extract concentrate. Methods for the concentration of fruit extracts include e.g. water removal by evaporation.

The concentration procedure will lead to an increase in the dry matter content in the liquid Acerola fruit extract. In consequence, the liquid Acerola fruit extract concentrate of the present invention has preferably a dry matter content of about at least 30% by weight, preferably at least 40% by weight, more preferably at least 50% by weight. In other words, the liquid Acerola fruit extract concentrate preferably has a dry matter content of from 20% to 80% by weight, preferably from 30% to 70% by weight, more preferably from 40% to 60% by weight. In a particularly preferred embodiment, the liquid Acerola fruit extract concentrate has a dry matter content of about 50% by weight.

The Acerola fruit has been considered one of the most important natural sources of vitamin C, with a vitamin C level of typically within about 1000 to 4500 mg per 100 g of pulp (Boulanger and Crouzet, 2001, Food Chem, 74:209-216), i.e. 1% by weight (w/w) to 4.5% by weight (w/w) vitamin C. Accordingly, the content of vitamin C in the concentrated liquid Acerola fruit extract (i.e. the liquid Acerola fruit extract concentrate) of the invention can be more than 17% by weight vitamin C. In one embodiment, the liquid Acerola fruit extract concentrate of the invention comprises e.g. more than 34% by weight vitamin C. The person skilled in the art is aware of methods to preserve the vitamin C content in Acerola plants, fruits and Acerola fruit extracts, for examples by avoiding the presence of oxygen, heavy metal irons, such as copper, silver and iron cations, alkaline pH and high temperatures.

It is preferred that the liquid Acerola fruit extract (concentrate) used in the method of the present invention comprises of from 1% to 40% by weight, preferably of from 4% to 30% by weight, more preferably from 8% to 20% by weight vitamin C. It is particularly preferred, that the liquid Acerola fruit extract (concentrate) comprises of from 8% to 20% by weight vitamin C. In other words, the liquid Acerola fruit extract (concentrate) comprises at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40% by weight or more vitamin C. It is particularly preferred that the liquid Acerola fruit extract (concentrate) comprises at least 5% by weight vitamin C. In a further preferred embodiment the liquid Acerola fruit extract (concentrate) comprises at least 10% by weight vitamin C. In still other words, the liquid Acerola fruit extract (concentrate) comprises about 1%, 2%, 3%, 4%, 5%, 6%, 7%, 8%, 9%, 10%, 11%, 12%, 13%, 14%, 15%, 16%, 17%, 18%, 19%, or 20% or more vitamin C by weight.

The high vitamin C levels in the liquid Acerola fruit extract used in the method of the present invention are conserved by spray drying and will, thus, lead to a high vitamin C level also in the final spray-dried composition. Thus, the spray-dried composition according to the present invention comprises preferably from 0.1% to 20% by weight, preferably from 0.5% to 15% by weight, more preferably from 1% to 10% by weight, still more preferably from 2% to 7% by weight vitamin C. It is particularly preferred, that the spray-dried composition comprises of from 2% to 7% by weight vitamin C. In other words, the spray-dried composition comprises at least 0.1%, 0.2%, 0.3%, 0.4%, 0.5%, 0.6%, 0.7%, 0.8%, 0.9%, 1%, 2%, 3%, 4%, 5%, or 6% by weight vitamin C. It is particularly preferred, that the spray-dried composition comprises at least 1% by weight vitamin C. In still other words, the spray-dried composition comprises about 0.05%, 0.1%, 0.2%, 0.3%, 0.4%, 0.5%, 0.6%, 0.7%, 0.8%, 0.9%, 1%, 2%, 3%, 4%, 5%, or 6% by weight vitamin C. In one embodiment, the spray-dried composition comprises about 4% by weight vitamin C.

Methods for the detection of the vitamin C content in fruit and fruit concentrate are well known in the art and e.g. described by Gensler et al., 1995, J Agric Food Chem, 43:2662-6. For example, one suitable and frequently used method is HPLC (high performance liquid chromatography).

The term "vitamin C" as used herein and known in the art refers to ascorbic acid and derivatives thereof that have equivalent effects, i.e. derivatives that have vitamin C activity, in mammals. Such derivatives are e.g. dehydroascorbic acid, ascorbate, calcium ascorbate, sodium ascorbate, and other salts of ascorbic acid. In other words, the term "vitamin C" includes all vitamin C vitamers. Vitamers of vitamin C have a vitamin C activity and generally also have similar molecular structures. Vitamin C vitamers are, e.g., ascorbic acid, dehydroascorbic acid, ascorbate, calcium ascorbate, sodium ascorbate, and other salts of ascorbic acid.

Vitamin C is a cofactor (electron donor) in several enzymatic reactions in mammalian metabolism. Among these enzymatic reactions are several that are involved in the synthesis of collagen. "Vitamin C activity" in the sense of the present invention should be understood as referring to vitamin C activity that is involved in the synthesis of collagen. Preferably, vitamin C activity means being able to serve as a cofactor for enzymes that are involved in the synthesis of collagen, more preferably, being able to serve as a cofactor for prolyl-3-hydroxylase, prolyl-4-hydroxylase, and lysyl hydroxylase. These enzymes are required for the hydroxylation of proline and lysine during the synthesis of collagen.

As mentioned before, the spray-dried compositions of and generated by the methods of the present invention provide advantageously very high levels of natural vitamin C. The uptake of synthetic vitamin C can therefore be avoided by use of the spray-dried compositions described herein. It is therefore preferred that the spray-dried composition comprises substantially only natural vitamin C. "Substantially only" is to be understood as a content of synthetic vitamin C of less than 5%, less than 4%, less than 3%, less than 2%, preferably less than 1% (by weight), more preferably less than 0.5% by weight, of the total vitamin C content. Preferably, the spray-dried composition has a content of synthetic vitamin C of less than 0.5% by weight of the total vitamin C content. In other words, the spray-dried composition has a content of synthetic vitamin C of from about 0% to about 5% by weight, preferably from about 0% to about 2.5% by weight, more preferably from about 0% to about 0.1% by weight of the total vitamin C content. However, it is particularly preferred that the liquid Acerola fruit extract and/or the spray-dried composition comprises no synthetic vitamin C.

Thus, preferably, no synthetic vitamin C is added, e.g. prior or simultaneously to the spray-drying in step b), during the method of the present invention. This means that neither pure synthetic vitamin C nor compositions comprising synthetic vitamin C are added.

"Natural vitamin C" is vitamin C of natural origin, in particular vitamin C from plants and in particular fruits. Sources of natural vitamin C other than Acerola fruits are, e.g. camu camu, oranges, guava, red or green pepper, kiwi, grapefruit, sprouts, tomatoes, strawberries, cantaloupe, and the like. It is, however, not necessary to include additional natural vitamin C in the spray-dried compositions because the natural vitamin C content in the Acerola fruit is sufficiently high. Therefore, the natural vitamin C comprised in the liquid Acerola fruit extract and the spray-dried composition described herein is preferably exclusively derived from Acerola. In further embodiments, at least 90% by weight, preferably at least 95% by weight, or more preferably at least 99% by weight of the natural vitamin C comprised in the spray-dried composition are derived from Acerola. In other words, from 90% to 100%, from 95% to 100%, or from 99% to 100% by weight of the natural vitamin C comprised in the spray-dried composition are derived from Acerola.

It is preferred that the liquid Acerola fruit extract (concentrate) used in the method of the present invention comprises of from 1% to 40% by weight, preferably of from 4% to 30% by weight, more preferably from 8% to 20% by weight natural vitamin C. In other words, the liquid Acerola fruit extract (concentrate) comprises at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40% by weight or more natural vitamin C. It is particularly preferred that the liquid Acerola fruit extract (concentrate) used in the method of the invention comprises at least 1% by weight natural vitamin C.

Preferably, no source of natural vitamin C other than the Acerola fruit extract is added prior or simultaneously to the spray-drying in step b) of the methods of the present invention. This means that neither pure, isolated natural vitamin C nor extracts or compositions comprising natural vitamin C are added in addition to the liquid Acerola fruit extract.

"Synthetic vitamin C" and "artificial vitamin C" are used synonymously and refer to vitamin C that has been artificially synthesized, i.e. synthesized by chemical, biochemical or biotechnological means. Methods for the discrimination between natural and artificial vitamin C are known in the art. For example, discrimination is possible by the conduction of stable isotope measurements of the vitamin C. This is based on the phenomenon that natural compounds, such as vitamin C, from the same origin have a characteristic isotope pattern which can be expressed e.g. as the carbon isotope ratio ($\delta^{13}$C value):

$$\delta^{13}C \text{ value} = (((R_{sample}) - (R_{standard})) / (R_{Standard})) * 1000(‰) \text{ wherein } R = [^{13}C]/[^{12}C], \text{ for PDB} = 0.011237.$$

PDB is the Pee Dee Belemnite standard, which originated from the fossil Belemnitella americana, which had an anomalously high $^{13}$C:$^{12}$C ratio and was established as $\delta^{13}$C of zero. It has been confirmed in the art that $\delta^{13}$C values, i.e. the carbon isotope ratio, of L-ascorbic acid are characteristic for their origin (Gensler et al., 1995, J Agric Food Chem, 43:2662-6). Therefore, a comparison of the $\delta^{13}$C value of a vitamin C preparation with the normal $\delta^{13}$C of a particular origin, indicates whether the vitamin C preparation stems partially, completely or not from this origin. Suitable methods for the determination of the isotope ratios are known in the art. For example, the isotope ratio can be determined by a method such as EA-IRMS (electron affinity-isotope ratio mass spectrometry).

It will be understood, that this method cannot only be applied to the carbon isotope ratio, but also to isotope ratios for other elements comprised in vitamin C. Alternatively or in addition to $\delta^{13}$C values, $\delta^{18}$O and/or $\delta^2$H values and/or isotopic patterns of vitamin C can be determined and compared to the values in Acerola fruit. In particular, a further method that is suitable for the discrimination between natural and synthetic vitamin C is the generation of isotopic patterns of vitamin C preparations.

An additional method that allows the discrimination between natural and artificial vitamin C, is the positional carbon 13 measurement. The synthesis of ascorbic acid from sugars in plants and in industrial processes includes different metabolic pathways, leading to a significant difference of carbon 13 at position 1 of the carbon atoms.

The person skilled in the art is aware that the isolation and analysis methods used should be isotope effect free. Any deviation from the determined standards will then indicate a contamination with artificial ascorbic acid. Knowledge of the geographical origin of the sample is not absolutely essential as the isotopic distribution between different components comprised in a preparation containing vitamin C is correlated. The analysis of the isotopic correlation of further compounds in the preparation can, thus, be used as an internal standard. The above described methods are suitable to distinguish natural from synthetic vitamin C as well as vitamin C from different sources.

The spray-dried compositions described herein and produced by the methods of the present invention comprise hydrolyzed collagen type II. Collagen is a characteristic structural protein that is mainly found and that is the main component in different connective tissues, such as skin, bone, cartilage, and the like, of animals and humans. 28 different types of collagen have been identified in vertebrates, including fibrillar, network, anchoring fibril, FACIT (fibril-associated collagen with interrupted triple helices), MACIT (membrane-associated collagen with interrupted triple helices), and MULTIPLEXIN (multiple triple helix domains and interruptions) collagens.

Characteristically, all collagen types form long fibrils that are made up of three polypeptide strands and are intertwined into a triple helix. This structure is caused by a particular pattern of amino acids in the polypeptide sequence, which comprises particularly high amounts of glycine (Gly), proline (Pro) and hydroxyproline (Hyp). For example, the average molecular weight of a single collagen type II molecule is well above 100 kDa.

The hydrolyzed collagen in the spray-dried compositions described herein and produced by the methods of the present invention is collagen type II. Collagen type II is the main component of cartilage, such as articular and hyaline cartilage. Hydrolyzed collagen type II may be used as a food supplement for improving the joint health. The hydrolyzed collagen in the spray-dried compositions described herein and produced by the methods of the present invention is therefore hydrolyzed collagen type II. Homo sapiens collagen type II, alpha 1, has a length of around 1400 amino acids, depending on the isoform, and a molecular weight of above 140 kDa. The term "collagen type II" as used herein comprises all collagen type II subtypes and isoforms.

Collagen can be a collagen derived from an animal selected from the group consisting of birds, mammals, and fish. For example, the collagen may be from a bird, such as from a bird selected from the group consisting of chicken, turkey, and geese. In a preferred embodiment, the collagen is chicken collagen. Alternatively, the collagen may be from a mammal e.g. selected from the group consisting of pigs, and cattle. Methods for the discrimination between collagens of different origins are known in the art and include, e.g. an analysis of the amino acid sequence. For example, fish collagen has a lower content of proline (Pro) and hydroxyproline (Hyp) and a lower thermal stability than mammalian collagen.

As mentioned above, collagen type II is mainly found in cartilage. Therefore, the fluid comprising hydrolyzed collagen type II according to step a) comprises cartilage-derived material comprising the hydrolyzed collagen type II. In a particularly preferred embodiment, the fluid comprising hydrolyzed collagen type II according to step a) consists substantially of cartilage-derived material comprising the hydrolyzed collagen type II and water, such as water having a pH between 4 and 8.

Cartilage can be cartilage from different parts of the animal, such as e.g. from sternum, fins, and scapula. In a particularly preferred embodiment, the cartilage is sternum cartilage. In a further preferred embodiment, the cartilage is chicken sternum cartilage. It is particularly preferred that the collagen is collagen type II derived from chicken sternum cartilage. Alternatively, the cartilage may also be from e.g. shark fins, or porcine or bovine scapula. Methods to obtain the cartilage from the animal body are well known in the art. For example, in case chicken sternal cartilage is used in the method of the present invention, fresh sternal cartilage is cut from chicken carcasses. All meat parts are removed from the cartilage and the cartilage is cut so that a space of about 2 mm is left from the bone, so as to avoid any bone fragments. The cartilage may be stored by freezing. By avoiding any bone fragments, a contamination of collagen type II with collagen types I and III originating from the bone can be minimized. Nevertheless it is noted that minor amounts of other collagen types in the fluids of the invention do not impair the ability of the hydrolyzed collagen type II to act as spray drying carrier for the Acerola fruit extract. In one embodiment, the fluid comprising collagen type II of step a) is substantially free of collagen type I and collagen type III.

"Cartilage-derived material" is to be understood as material that has been obtained from cartilage by cuttering, shredding, chopping, grinding, mincing, diluting, and/or dissolving the cartilage and, optionally, concentrating the resulting solution, and the like. Furthermore, the cartilage-derived material according to the invention undergoes a hydrolyzation treatment, as described elsewhere herein, before use in the mixing step a). This also means that the cartilage-derived material comprises preferably essentially all components that were originally comprised in the cartilage, such as *inter alia* collagen type II, chondroitin sulfate and hyaluronic acid. The cartilage-derived material is, hence, a mixture of different compounds comprised in the cartilage from which the material was derived.

In one embodiment of the invention, the cartilage-derived material has been prepared (obtained) from cartilage by hydrolysis of the cartilage. The cartilage can be hydrolyzed as a whole or cut into smaller pieces before hydrolysis (such as pieces with an approximate diameter of from 1 mm to 5 cm, preferably from 2.5 mm to 2.5 cm, more preferably from 5 mm to 1 cm). Hydrolysis of the cartilage may be by enzymatic hydrolysis, by hydrolysis with heat, incubaction with bases or acids. In a preferred embodiment, hydrolysis of the cartilage is by incubation with a protease, such as by incubation with one or more proteases selected from the group consisting of papain, ficin, bromelain, and endoproteases, such as alcalase. In one embodiment, the cartilage is suspended in an aqueous solution, preferably water, before hydrolysis. Suspension in the aqueous solution may be for at least 10 min, 20 min, 30 min, 40 min, 50 min, or more. The cartilage-derived material may then be filtered, e.g. through diatomaceous earth, before use in step a) of the method of the invention. In a preferred embodiment, the cartilage-derived material has been prepared (obtained) from cartilage by hydrolysis of the cartilage and subsequent filtration, wherein the filtrate is the cartilage-derived material.

Preferably, the spray-dried compositions according to the present invention consist substantially of an Acerola fruit extract, cartilage-derived material comprising hydrolyzed collagen type II and chondroitin sulfate. In this embodiment, the compositions do not comprise additional compounds or ingredients, thereby providing a maximum concentration of the beneficial compounds comprised in the Acerola fruit extract and in the cartilage-derived material comprising the hydrolyzed collagen type II. This does not exclude that the spray-dried compositions may contain minor amounts of residual thermally inactivated proteolytic enzymes (as a result of the hydrolysis process).

"Hydrolyzed" collagen in the sense of the present invention relates to collagen that has been hydrolyzed, for example enzymatically hydrolyzed, i.e. proteolytically cleaved, or hydrolyzed by heat, incubation with bases or acids, or by other methods. Preferably, the collagen is hydrolyzed by proteolytic enzymes, such as endoproteases, exopeptidases, alkaline and neutral endoproteases. Methods and enzymes suitable for the enzymatic hydrolyzation as well as corresponding reaction conditions are well known in the art and may be performed in the context of the present invention prior to step a) to prepare a fluid comprising hydrolyzed collagen. A suitable method for the preparation of hydrolyzed collagen compositions is, e.g. described in US 6,025,327 and in particular the method for preparation of hydrolyzed collagen type II from chicken sternal cartilage that is described therein. In short, the cartilage comprising type II collagen is cuttered and suspended in aqueous fluid, such as water, for about one hour at approximately 35°C. The aqueous fluid has a pH of between about 4 and 8, preferably about 6.5. Subsequently, the cartilage-comprising fluid is incubated with one or more proteases for about 2 to 10 hours at about 35°C to 55°C at a pH of between 4 and 8.

Suitable proteases that are capable to hydrolyze collagen are e.g. papain, ficin, bromelain, and endoproteases, such as alcalase. Thus, the hydrolyzed collagen is preferably a hydrolyzate selected from the group consisting of a papain hydrolyzate, a ficin hydrolyzate, a bromelain hydrolyzate, an endoprotease hydrolyzate or a mixture thereof. The skilled person is able to adapt the reaction conditions in a manner that ensures that the desired average molecular weight of the collagen is obtained.

After hydrolyzation, the length and, accordingly, the molecular weight of the collagen molecules have been considerably reduced. The shortened collagen peptides can be efficiently taken up and metabolized by the human body. The hydrolyzed collagen according to the present invention preferably has an average molecular weight of from 0.1 to 13.5 kDa, more preferably of from 0.5 to 5 kDa, most preferably of from 1 to 2.5 kDa. In one embodiment, the hydrolyzed collagen has an average molecular weight of from 0.5 to 5 kDa. It is particularly preferred that the average molecular weight is from 1 to 2.5 kDa, preferably from 1.5 to 2.5. In a similar embodiment, the average molecular weight is from 1 to 1.5 kDa.

The fluid comprising hydrolyzed collagen that is used in the methods of the present invention may e.g. comprise from 5 % to 50 %, from 8% to 45%, from 10% to 40%, from 15% to 35%, from 12% to 30%, from 20% to 30% (by weight) hydrolyzed collagen. In a preferred embodiment, the fluid comprises from 12% to 30% by weight hydrolyzed collagen. In another embodiment, the fluid comprises from 20% to 30% by weight hydrolyzed collagen. These percentages refer to the dry matter content of hydrolyzed collagen. In a particularly preferred embodiment, the fluid comprising hydrolyzed collagen comprises about 25% by weight hydrolyzed collagen. These percentages refer to the content of hydrolyzed collagen type II. This means e.g. that the fluid comprises from 12% to 30% by weight hydrolyzed collagen type II, preferably, from 20% to 30% collagen type II.

As outlined elsewhere herein, the cartilage-derived material for use in the method of the present invention comprises only minor amounts of other collagens while its main ingredient is collagen type II. Therefore, similar values as defined for the hydrolyzed collagen above, apply also to the content of cartilage-derived material in the used fluid. Thus, the fluid may e.g. comprise from 5% to 50 %, from 8% to 45%, from 10% to 40%, from 15% to 35%, from 12% to 30%, from 20% to 30% (by weight) cartilage-derived material. These percentages 12% to 30%, from 20% to 30% (by weight) cartilage-derived material. These percentages refer to the dry matter content of cartilage-derived material. In a preferred embodiment, the fluid comprises from 15% to 35% by weight, more preferably from 20% to 30% by weight cartilage-derived material. In a particularly preferred embodiment, the fluid comprises about 25% by weight cartilage-derived material. However, the fluid may also comprise higher levels of cartilage derived material, such as 20% to 50% by weight.

In consequence of this concentration in the fluid, the spray-dried compositions according to the present invention have e.g. a concentration of hydrolyzed collagen type II of from 40% to 90% by weight. In other words, the hydrolyzed collagen is present in the spray-dried compositions in an amount between 40g/100g to 90g/100g. In further embodiments, the spray-dried compositions comprise between 45% and 85%, preferably between 50% and 80%, more preferably between 55% and 75% hydrolyzed collagen. This means that the spray-dried compositions comprise at least 40%, 45%, 50%, 55%, 60%, 65%, 70%, 75%, 80%, 85%, or 90% (by weight) hydrolyzed collagen. In a preferred embodiment, the spray-dried compositions comprise at least 40% hydrolyzed collagen. In a farther preferred embodiment, the spray-dried compositions comprise at least 60% hydrolyzed collagen. These percentages refer to the content of hydrolyzed collagen type II. For example, the spray-dried compositions can comprise at least 40% hydrolyzed collagen type II.

The "fluid comprising hydrolyzed collagen" for use in the method of the invention has a dry matter content of preferably between 20% and 50% by weight. The fluid according to the invention can be e.g. a solution, suspension, dispersion, or a mixture thereof. In a preferred embodiment, the fluid is a solution. However, the term "solution" should not be understood as excluding solutions, in which the ingredients (such as hydrolyzed collagen or cartilage-derived material) are not completely dissolved. Suspensions comprising cartilage-derived material comprising hydrolyzed collagen type II are used in step a) of the method of the present invention.

Preferably, the fluid is an aqueous fluid. It is preferred that the fluid has a pH of between about 3 and 8, preferably of between 4 and 7, more preferably about 5.

Obtaining the collagen from cartilage material, such as chicken sternum cartilage, provides the further advantage that the final product is also high in mucopolysaccharides, particularly in chondroitin sulfate. Thus, the fluid will comprise at least 5 % by weight, more preferably at least 10% by weight, most preferably at least 20% by weight chrondroitin sulfate. Thus, according to the invention, the fluid comprises at least 5% by weight chrondroitin sulfate. In other words, the fluid comprises preferably from 5% to 70% by weight, more preferably from 10% to 50% by weight, most preferably from 20% to 40% by weight chondroitin sulfate. To put it another way, the fluid comprises at least 50 g/l, and more preferably at least 100 g/l chondroitin sulfate, for example from 50 g/l to 300 g/l.

In consequence of this concentration in the fluid, the spray-dried compositions according to the present invention have e.g. a concentration of chondroitin sulfate of from 10% to 30%, preferably from 15% to 25% by weight. In one embodiment, the spray-dried compositions have a concentration of chondroitin sulfate of from 10% to 30% by weight. In other words, the spray-dried compositions comprise at least 10%, preferably at least 15%, more preferably at least 20% by weight chondroitin sulfate. In one embodiment, thus, the spray-dried compositions comprise at least 10% by weight chondroitin sulfate.

The method according to claim 1 for preparing a spray-dried composition comprises a step (a) of mixing a liquid Acerola fruit extract obtained by pressing of an Acerola fruit and a fluid comprising cartilage-derived material comprising hydrolyzed collagen type II and at least 5% by weight of the fluid chondroitin sulfate. Mixing includes the addition of the fruit extract to the fluid comprising hydrolyzed collagen or *vice versa.* Mixing further includes intermixing of extract and fluid comprising hydrolyzed collagen which can be by any suitable method known in the art, such as by diffusion, stirring, rolling or shaking.

Depending on the desired properties for the spray-dried mixture, such as vitamin C content or content of hydrolyzed collagen, the liquid Acerola fruit extract and the fluid comprising hydrolyzed collagen can be mixed in different ratios. For instance, it has been shown in the examples described herein that the yield of the spray drying procedure is highest in a certain mixing ratio. Therefore, in a preferred embodiment, the liquid Acerola fruit extract and the fluid comprising hydrolyzed collagen are mixed in a ratio of from 0.1:100 to 65:100, preferably of from 1:100 to 55:100, or more preferably from 10:100 to 30:100, such as e.g. about 40:100, in step a). In one embodiment, the liquid Acerola fruit extract and the fluid comprising hydrolyzed collagen are mixed in a ratio of from 1:100 to 55:100 in step a). In a particularly preferred embodiment, the liquid Acerola fruit extract and the fluid comprising hydrolyzed collagen are mixed in a ratio of from 10:100 to 30:100 in step a). These ratios are total weight ratios.

Expressed with regard to the solid matter contents of the liquid Acerola fruit extract and the fluid comprising hydrolyzed collagen this means that the liquid Acerola fruit extract and the fluid comprising hydrolyzed collagen are mixed in a ratio (solid matter:solid matter) of from 5:100 to 70:100, preferably from 1:100 to 65:100, more preferably from 10:100 to 60:100, such as about 14:100, about 28:100 or about 55:100 (compare example section elsewhere herein). Thus, in one embodiment the liquid Acerola fruit extract and the fluid comprising hydrolyzed collagen are mixed in a solid matter ratio of from 1:100 to 65:100 in step a). In a particularly preferred embodiment, the liquid Acerola fruit extract and the fluid comprising hydrolyzed collagen are mixed in a solid matter ratio of from 10:100 to 60:100 in step a). "Solid matter content" (otherwise known as "dry matter" or "dry weight") is a measure of the mass when completely dried. This means that the water has been essentially completely removed when the solid matter content is measured. Methods for the determination of the solid matter content of fruit extracts and other liquids are known in the art.

Notably, the mixing ratio also affects the vitamin C content in the final product. Therefore, in another embodiment, the liquid Acerola fruit extract and the fluid comprising hydrolyzed collagen are mixed in a ratio of about 10:100. This way, a final vitamin C concentration of about 4% by weight is achieved. As can be seen, the optimal mixing ratio differs depending on the desired outcome, e.g. whether a high yield and/or a particular vitamin C concentration is desired.

The liquid Acerola fruit extract and/or the fluid comprising hydrolyzed collagen may be filtered before mixing in step a). The person skilled in the art is aware of suitable filtering methods, such as sterile filtration methods. For example, the hydrolysate can be filtered through diatomaceous earth or by vacuum filtration.

Finally, the method of the present invention comprises a step (b) of spray-drying the mixture. Suitable spray-drying methods and spray-drying apparatuses are known in the art and routinely used (compare e.g. Patel et al., 2009, Ind J Sci Techn, 2(10):44-47). One example for a suitable spray-drying apparatus is the standard model 6-12 (European SprayDry Technologies, Essex, UK), which can, e.g. be equipped with a 60-68-pressure nozzle (Spraying Systems Co., Hamburg, Germany). Spray drying involves 3 fundamental processes; liquid atomisation, evaporation and separation/collection of the dried product. The material to be dried is sprayed, in the form of small droplets, into a vertical drying chamber. At the same time a large volume of hot gas (air) is fed into the chamber to evaporate the liquid content of the sample. Heat and mass transfer takes place as the dispersed droplets are in direct contact with the hot air. The person skilled in the art is able to choose suitable inlet and outlet temperatures, such as e.g. 180°C and 80°C, respectively. These temperatures have been used in the examples described elsewhere herein and are, thus, preferred in embodiments of the invention. Thus, preferably, spray drying in step b) is carried out with an inlet temperature of from 160°C to 200°C, more preferably of from 170°C to 190°C. Using an inlet temperature of about 180°C in step b) is particularly preferred. Also, spray drying in step b) is preferably carried out with an outlet temperature of from 60°C to 100°C, more preferably from 70°C to 90°C. Using an outlet temperature of about 80°C in the spray drying during step b) is particularly preferred. On completion of drying, the hot air and solids are separated. Dried product is transferred from the base of the chamber and conveyed to a high efficiency cyclone where the powder and drying gas are separated. In a preferred embodiment, the mixture is spray-dried using a size 60 to 68 pressure nozzle. The final particle size and mesh are adjusted to 0.46 g/cm$^3$. Thereby a yield of a fine powder is ensured.

Preferably, no additional excipients (such as suspending agents, dispersing or wetting agents; i.e. substances other than the Acerola fruit extract and the fluid comprising hydrolyzed cartilage-derived collagen type II and chondroitin sulfate) are added before, during, or after step a).

In particular, as the present invention surprisingly identified hydrolyzed collagen as a suitable spray-drying carrier for Acerola fruit extract, it is not necessary to add further spray drying carriers (also known as "drying aids") normally used in the art, such as an amphiphatic carrier, maltodextrin, dextrin, oxidized starch, lactose, fumed silica (such as Aerosil® type), cashew tree gum, Arabic gum or the like. Therefore, in a preferred embodiment, no spray-drying carrier other than the hydrolyzed collagen is added prior or simultaneously to the spray-drying in step b). In other words, it is preferred that no spray-drying carrier other than the cartilage-derived material is added prior or simultaneously to the spray-drying in step b). Expressed differently, no spray-drying carrier other than compounds comprised in the cartilage-derived material or potentially comprised in Acerola fruit extract is added prior or simultaneously to the spray-drying in step b). For example, no amphiphatic carrier, no maltodextrine, dextrine, oxidized starch, lactose, fumed silica, cashew tree gum, or Arabic gum will be added prior or simultaneously to the spray-drying in step b).

In the art, polysaccharides, such as maltodextrin, dextrin, oxidized starch, lactose, and the like were often used as spray-drying carriers. Thus, in a particularly preferred embodiment the term "spray-drying carrier" is to be understood as "polysaccharide". In this embodiment, no polysaccharide (other than polysaccharides potentially comprised in the cartilage-derived material or the Acerola fruit extract) is added prior or simultaneously to the spray-drying in step b). In particular, no maltodextrin, lactose and/or oxidized starch are added prior or simultaneously to the spray-drying in step b).

In accordance with the above the invention provides a spray-dried composition, comprising or consisting of (a) the cartilage-derived material which comprises the hydrolyzed collagen type II and chondroitin sulfate; and (b) the Acerola fruit extract. It will be understood that the spray-dried composition (such as the composition prepared by the method described herein) is a powder, such as a powder having particle sizes described elsewhere herein. In a preferred embodiment, the spray-dried composition consists of (a) the cartilage-derived material which comprises hydrolyzed collagen type II and chondroitin sulfate; and (b) the Acerola fruit extract. As described before, the term "Acerola fruit extract" refers to a mixture of all substances comprised in Acerola fruit juice and does not refer to isolated vitamin C alone.

Preferably, the spray-dried composition comprises no spray-drying carrier other than compounds comprised in the cartilage-derived material or (potentially) comprised in the Acerola fruit extract. In particular, said spray-dried composition comprises or consists of (a) the cartilage-derived material which comprises hydrolyzed collagen type II and chondroitin sulfate; and (b) the Acerola fruit extract; and comprises no maltodextrine, oxidized starch and/or lactose. It is further preferred that the spray-dried composition comprises no spray-drying carrier selected from the group consisting of maltodextrine, dextrine, oxidized starch, lactose, fumed silica (such as Aerosil® type), cashew tree gum, and/or Arabic gum, and the like. Further, it is preferred that the composition comprises no amphiphatic carrier. In a preferred embodiment, the composition comprises no polysaccharide that does not stem from the fluid comprising hydrolyzed collagen or the liquid Acerola fruit extract. In particular, the composition does not comprise maltodextrin, lactose and/or oxidized starch.

Desired concentrations of the different components of the spray-dried compositions according to the invention are outlined in detail elsewhere herein. Nevertheless, it is emphasized that it is preferred that the composition comprises of from 20% to 50% by weight hydrolyzed collagen type II. The composition further comprises at least 5% by weight of chrondroitin sulfate, preferably at least 10% by weight, at least 15% by weight or at least 20% by weight chrondroitin sulfate.

As it is one advantage of the invention that it is able to provide so-called clean-label products with a high content of natural vitamin C, in certain embodiments, the spray-dried composition (a) comprises at least 0,1 % by weight natural vitamin C; (b) comprises no source of natural vitamin C other than the Acerola fruit extract, such as a Cranberry extract; and/or (c) comprises no synthetic vitamin C. Preferably, the spray-dried composition (a) comprises at least 0,1 % by weight natural vitamin C; and (c) comprises no synthetic vitamin C. More preferably, the spray-dried composition (a) comprises at least 1 % by weight natural vitamin C; and (c) comprises no synthetic vitamin C. This means that in one particularly preferred embodiment, the spray-dried composition comprises at least 0,1 % by weight natural vitamin C, preferably at least 1 % by weight, more preferably at least 3 % by weight. In other words, the spray-dried composition comprises preferably from 0.1% to 40% by weight natural vitamin C, more preferably from 1% to 20% by weight natural vitamin C. Other preferred vitamin C contents have been outlined elsewhere herein. In a further preferred embodiment, the spray-dried composition comprises no source of natural vitamin C other than the Acerola fruit extract. For example, the composition does not comprise other fruit extracts, such as a Cranberry extract. Preferably, the spray-dried composition comprises no synthetic vitamin C.

In another aspect, the invention relates to a food supplement according to claim 10 comprising the spray-dried composition described herein. A food supplement is an edible composition comprising at least the spray-dried composition of the present invention. The food supplement is suitable for ingestion as a food stuff, a nutritional supplement, an animal feed supplement, or a nutraceutical.

The food supplement is in solid form, such as a powder, granule, tablet and the like. Thus, in a preferred embodiment, the food supplement is a powder. In another embodiment, the food supplement is a tablet. Further, the food supplement may also be in liquid form, such as an aqueous or oil solution, emulsion, and the like, such as in a capsule or a drink. The spray-dried composition (which is water-soluble) can be dissolved in the liquid food supplement. The food supplement may also have the form of jelly, snacks or nutritional bars.

Disclosed is a non-therapeutic use of a spray-dried composition described herein or a food supplement described herein to improve cartilage and joint health in an individual. The spray-dried compositions are preferably for use as a food supplement, more preferably for use as a food supplement to improve cartilage and joint health in an individual. Methods for the assessment of cartilage and joint health are well known in the art. An improvement in cartilage and joint health is for example often associated with an increase in mobility or flexibility of the joint. Such an improvement can be subjectively felt by the individual which received the compositions itself.

Further provided is a non-therapeutic use of a spray-dried composition described herein or a food supplement described herein to reduce skin wrinkles, in particular sun-induced skin-wrinkles, and/or to increase skin flexibility. Thus, the spray-dried compositions can be for use as a food supplement for use in the reduction of skin wrinkles, in particular sun-induced skin-wrinkles, and/or to increase skin flexibility.

Also a non-therapeutic use of a spray-dried composition described herein or a food supplement described herein as a prophylactic agent in a healthy individual is provided. Thus the spray-dried compositions can be for use as a food supplement for use as a prophylactic agent in a healthy individual. A "healthy" individual in the sense of the present invention is one that does not visibly or noticeably suffer from a disease or condition that can be improved through the supplementation of hydrolyzed collagen.

For these non-therapeutic uses, the spray-dried compositions or food supplements are preferably taken orally. Nevertheless, enteral and intragastric administration is also possible. In all non-therapeutic uses, the administration is preferably to a substantially healthy individual.

Preferably, in these non-therapeutic uses, the spray-dried compositions and food supplements are administered in daily doses of hydrolyzed collagen from 500 mg to 10 000 mg, more preferably of from 1000 mg to 5000 mg. In a particularly preferred embodiment, the spray-dried compositions and food supplements are administered in daily doses of hydrolyzed collagen of from 1 g to 5 g.

In another aspect, the invention as set forth in claim 9 is concerned with the spray-dried compositions described herein for use in a method of treating or preventing connective tissue disorders or diseases which would benefit from increased synthesis of cartilage.

Additionally, in a further aspect, the invention as set forth in claim 9 relates to a method for treating or preventing connective tissue disorders or diseases which would benefit from increased synthesis of cartilage.

Connective tissue disorders or diseases which would benefit from increased synthesis of cartilage are e.g. selected from the group consisting of degenerative joint diseases (such as rheumatoid arthritis), joint defects, osteoarthritis, polychondritis, vascular disease, cartilage injuries, autoimmune diseases involving connective tissue autoantibodies (such as rheumatoid arthritis), progressive myopia, Menier's disease and any other connective tissue disorder which would benefit from increased synthesis of cartilage. In a particularly preferred embodiment, the spray-dried compositions are for use in a method of treating or preventing rheumatoid arthritis.

Administration may be by any means known in the art, for example, by oral, enteral or intragastric administration. Oral administration is particularly preferred.

The spray-dried composition will be administered in a pharmaceutically effective amount. A "pharmaceutically effective amount" is an amount that is useful in the treatment or prevention of a disease or condition. Methods for the assessment of an improvement in joint comfort and the like are well known in the art.

Administration may be in a liquid of the patient's choice (as the spray-dried composition is water-soluble) or in tablet form. Before administration, the spray-dried composition may be dissolved in the liquid solution or suspension or the composition may be used with physiologically appropriate stabilizing agents, excipients, bulking agents, surfactants, or combinations thereof. Examples of suitable excipients include, but are not limited to, buffers, viscosity modifiers, or other therapeutically inactive but functional additives.

For example, the dose of the spray-dried composition may be selected in a manner that ensures a daily dose of 2 g collagen (type II) hydrolysate. Efficacy of a 10 g dose (without Acerola fruit extract) was demonstrated e.g. by Clark et al. (2008, Curr Med Res a Opin, 24(5): 1485-96). In contrast to the isolated collagen hydrolysate used therein, the present invention provides compositions that comprise several compounds that are crucial for cartilage regeneration. Moreover, the compounds, in particular the hydrolyzed collagen (type II) and vitamin C, are already present in an ideal mixing ratio. Advantageously, the present spray-dried composition is therefore more effective than standard collagen hydrolysates and may, thus, be administered e.g. in the aforementioned lower dose (2 g collagen (type II) hydrolysate/day). Administration may be performed e.g. for 24 consecutive weeks, such as demonstrated by Clark et al. (2008, Curr Med Res a Opin, 24(5): 1485-96), or for 6 months, such as demonstrated by Benito-Ruiz et al. (2009, Int J Food Sci Nutr, 60(S2): 99-113). It has been shown in the art that patients with a low meat intake, and hence a relatively low intake of dietary collagen, benefit to a greater extent from the administration of hydrolyzed collagen than patients with a high meat intake. Therefore, in a preferred embodiment, the patient or healthy person receiving the treatment or taking the food supplement is a person that has a low or no meat intake at the time of ingesting the spray-dried compositions.

**EXAMPLES**

The present invention is illustrated by the following examples.

**Example 1: Acerola fruit extract**

Acerola Juice Concentrate (BMgLC-18-219) was purchased from NutriBotanica (Brazil). According to the product information leaflet, the concentrate comprised more than 18 mg/ 100 ml vitamin C, and had a pH of between 2.8 and 4.5. Odor, taste and appearance were assessed by organoleptic testing and classified as "characteristic".

**Example 2: Preparation of hydrolyzed chicken sternum cartilage**

Hydrolyzed chicken sternum cartilage was prepared as described in US Patent No. 6,025,327. In short, fresh chicken sternal cartilage was cut not less than about 2 mm from the bone and the cartilage was suspended in water having a pH between 4 and 8. Then, the cartilage solution was enzymatically hydrolyzed. The hydrolysate was sterilized (by heating to 95°C for about 30 minutes), and filtered in a frame filter press with overhang filter cloth (polypropylene, Marsyntex). For a second, subsequent filtration step, filter layers with a separation limit of 6.0 to 15.0 $\mu$m were used (type SK200, StrassBurger Filter). Subsequently, the hydrolysate was concentrated to a concentration of 20 to 50% (by weight) solid matter content under vacuum. The hydrolyzed chicken sternum cartilage comprises about 60% (by weight, % of dry weight) collagen type II.

**Example 3: Preparation of a spray-dried mixture of hydrolyzed chicken sternum cartilage and Acerola fruit**

Hydrolyzed sternal chicken cartilage concentrate and Acerola concentrate, prepared as described above, were mixed in different ratios (100:10, 100:20, 100:40 and 100:70, respectively). Then, the mixture was spray-dried using a size 60-68-pressure nozzle (Spraying Systems Co., Hamburg, Germany), inlet temperature of about 180°C, outlet temperature of about 80°C, in a simple stage concurrent spray drying system (standard model 6-12, European SprayDry Technologies, Essex, UK).

During the spray drying process difficulties arose due to bonding and/or sticking because of the Acerola concentrate. It was found that the ratio of Acerola concentrate and collagen hydrolysate is important in order to achieve a good result, i.e. achieve the highest yield with the desired vitamin C content.

*1. Production Trial*

100 kg hydrolyzed sternal chicken cartilage and 10 kg Acerola juice concentrate were homogenized and spray-dried. The inlet temperature was approximately 180°C, the outlet temperature approximately 80°C. The yield of the final product was 32 kg.

*2. Production Trial*

100 kg hydrolyzed sternal chicken cartilage and 20 kg Acerola juice concentrate were homogenized and spray-dried. The inlet temperature was approximately 180°C, the outlet temperature approximately 80°C. The yield of the final product was 34 kg.

*3. Production Trial*

100 kg hydrolyzed sternal chicken cartilage and 40 kg Acerola juice concentrate were homogenized and spray-dried. The inlet temperature was approximately 180°C, the outlet temperature approximately 80°C. The yield of the final product was 42 kg.

*4. Production Trial*

100 kg hydrolyzed sternal chicken cartilage and 70 kg Acerola juice concentrate were homogenized and spray-dried. The inlet temperature was approximately 180°C, the outlet temperature approximately 80°C. This trial had to be stopped because of bonding during drying process. The yield of the final product was 2,8 kg .

The first production trial using a mixture of 100 kg hydrolyzed sternal chicken cartilage and 10 kg Acerola showed the best results regarding the vitamin C content and, e.g., the fineness of the powder even though the 2nd and 3rd production trial also gave good results.

**Example 4: Analysis of spray-dried mixture of hydrolyzed sternal chicken cartilage and Acerola fruit concentrate obtained from production trial 1**

After spray drying the mixture of 100 kg hydrolyzed collagen and 10 kg Acerola, a fine powder was received with results in accordance with the desired specification, i.e. having the desired Vitamin C content. The product specification can be found in Table 1.

**Table 1:** Product specification for spray-dried composition comprising cartilage-derived collagen type II and Acerola fruit extract. (Abbreviations: Ph.Eur.: Pharmacopoea Europaea; mod.: modified; o.d.b.: on dry base; USP: US Pharmacopeia; % = % by weight)

| Article No.: | KN-30-5-1-300 | |
|---|---|---|
| Raw materials: | Sternal chicken cartilage, Acerola | |
| **Test-Parameter** | **Test-Method** | **Specification** |
| Description: | visual | amorphous powder |
| Colour: | visual | beige |
| Odour: | organoleptic | neutral |
| Solubility: (1 % by weight in H2O) | visual | water-soluble |
| Ash: | A400 / Ph. Eur. 2.2.16 mod. | max. 10,0 % o.d.b. |
| Lipids: | F100 | max. 2,0 % o.d.b. |
| Nitrogen (Kjeldahl): | S200 / Ph. Eur. 2.5.9 | min. 8 % o.d.b. |
| Loss on drying (2h, T = 105°C) : | T400 / Ph. Eur. 2.2.32 | max. 5,0 % |
| pH (1% in H2O): | P400 / Ph. Eur. 2.2.3 mod. | 4,0 - 6,0 |
| Bulk density: | Ph. Eur. 2.2.42 | 0,40 - 0,55 g/ml |
| Tapped density: | Ph. Eur. 2.2.42 | 0,50 - 0,65 g/ml |
| Chondroitin Sulfate: | Based on USP 32 | min. 16% |
| Vitamin C: | HPLC | ~ 4% |

It can be seen that the content of chondroitin sulfate was within desired specifications, i.e. at least 16% by weight. The vitamin C content was approximately 4% by weight. Further analysis of the mixture is shown in Tables 2 to 4:

**Table 2:** Heavy metal content in the spray-dried mixture obtained from production trial 1 (i.e. 100 kg hydrolyzed collagen and 10 kg Acerola fruit extract). The determination was made by ICP-OES after microwave pressure revealing. Batch no.: FD-040530172.

| **Heavy metals** | **mg/kg** |
|---|---|
| Arsenic | 0,02 |
| Cadmium | <0,005 |
| Chrome | <0,003 |
| Mercury | <0,03 |
| Tin | <0,03 |
| Lead | <0,01 |
| Antimony | <0,007 |

**Table 3:** Amino acid content in the spray-dried mixture obtained from production trial 1. The amino acid analysis was done with an Amino Acid Analyzer, LC 3000 (Eppendorf-Biotronik), using cation exchange techniques with a five-step gradient and post-column derivatisation with ninhydrine and photometric detection at 440nm/570nm. Batch no.: FD-040530172

| Amino acids | µg/mg | |
|---|---|---|
| Taurine | 0,78 | |
| Hydroxyproline | 35,65 | |
| Asparagine | 38,00 | |
| Threonine | 17,50 | |
| Serine | 14,53 | |
| Glutamic acid | 70,68 | |
| Proline | 47,99 | |
| Glycine | 84,30 | |
| Alanine | 38,88 | |
| Cysteine | 2,67 | |
| Valine | 18,50 | |
| Methionine | 8,91 | |
| Isoleucine | 13,54 | |
| Leucine | 26,60 | |
| Tyrosine | 7,03 | |
| Phenylalanine | 14,52 | |
| β-Alanine | 8,27 | |
| Tryptophan | 0,35 | |
| Histidine | 10,36 | |
| 3-Methyl-Histidine | 11,93 | |
| Hydroxylysine | 7,85 | |
| Lysine | 21,95 | |
| $NH_3$ | 11,50 | |
| Arginine | 39,19 | |

**Table 4:** Content of different kinds of sugar in the final product, i.e. the spray-dried mixture obtained from production trial 1. Method: HPLC; Batch no.: FD-040530172.

| Fructose | 1,4 | g/100 g |
|---|---|---|
| Glucose | 4,3 | g/100 g |
| Saccharose | 1,3 | g/100 g |
| Maltose | <0,5 | g/100 g |
| Lactose | <0,5 | g/100 g |

**Claims**

1. Method for preparing a spray-dried composition comprising an Acerola fruit extract and hydrolyzed collagen, the

EP 3 053 458 B1

method comprising the steps of

(a) mixing a liquid Acerola fruit extract obtained by pressing of an Acerola fruit and a fluid comprising cartilage-derived material comprising hydrolyzed collagen type II and at least 5 % by weight of the fluid chondroitin sulfate;
(b) spray-drying the mixture.

2. Method according to claim 1, wherein no synthetic vitamin C is added prior or simultaneously to the spray-drying in step b).

3. Method according to claim 1 or 2, wherein the fluid comprises of from 20% to 50% by weight hydrolyzed collagen.

4. Method according to any of claims 1 to 3, wherein the liquid Acerola fruit extract and the fluid comprising hydrolyzed collagen are mixed in a ratio of from 5:100 to 70:100 with regard to the solid matter contents of the extract and the fluid in step a).

5. Spray-dried composition obtainable according to the method of any of claims 1 to 4, comprising

(a) cartilage-derived material comprising hydrolyzed collagen type II, chondroitin sulfate; and
(b) Acerola fruit extract comprising vitamin C and all different compounds that are comprised in Acerola fruit juice.

6. Spray-dried composition according to claim 5, wherein the composition comprises no spray-drying carrier other than hydrolyzed collagen.

7. Spray-dried composition according to claim 5 or 6, wherein the composition comprises no spray-drying carrier selected from the group consisting of maltodextrine, dextrine, oxidized starch, lactose, fumed silica, cashew tree gum, and Arabic gum.

8. Spray-dried composition according to any of claims 5 to 7, wherein the composition

(a) comprises at least 0,1 % by weight natural vitamin C;
(b) comprises no source of natural vitamin C other than the Acerola fruit extract; and/or
(c) comprises no synthetic vitamin C.

9. Spray-dried composition according to any of claims 5 to 8, for use in a method of treating or preventing a disease which would benefit from increased synthesis of cartilage, such as arthritis.

10. Food supplement comprising spray-dried composition according to any of claims 5 to 8, wherein the food supplement is in solid form such as a powder, a granule or a tablet.

**Patentansprüche**

1. Verfahren zur Herstellung einer sprühgetrockneten Zusammensetzung, die Acerola-Fruchtextrakt und hydrolysiertes Kollagen umfasst, wobei das Verfahren Schritte umfasst, bei denen man:

(a) einen flüssigen Acerola-Fruchtextrakt, der durch Ausdrücken einer Acerola-Frucht erhalten wurde, und eine Flüssigkeit mischt, wobei die Flüssigkeit aus Knorpel gewonnenes Material umfasst, das hydrolysiertes Typ II Kollagen und Chondroitinsulfat in einer Konzentration von mindestens 5 Gew.-% der Flüssigkeit enthält;
(b) die Mischung sprühtrocknet.

2. Verfahren gemäß Anspruch 1, **dadurch gekennzeichnet, dass** vor oder gleichzeitig zur Sprühtrocknung in Schritt b) kein synthetisches Vitamin C zugesetzt wird.

3. Verfahren gemäß Anspruch 1 oder 2, bei dem die Flüssigkeit 20 bis 50 Gew.-% hydrolysiertes Kollagen umfasst.

4. Verfahren gemäß einem der Ansprüche 1 bis 3, bei dem der flüssige Acerola-Fruchtextrakt und die Flüssigkeit, die hydrolysiertes Kollagen enthält, in einem Verhältnis von 5:100 bis 70:100 in Bezug auf den Feststoffgehalt des Extrakts und der Flüssigkeit in Schritt a) vermischt werden.

**5.** Sprühgetrocknete Zusammensetzung, die nach dem Verfahren gemäß einem der Ansprüche 1 bis 4 erhältlich ist, umfassend:

(a) aus Knorpel gewonnenes Material, das hydrolysiertes Typ II Kollagen und Chondroitinsulfat umfasst; und
(b) Acerola-Fruchtextrakt, umfassend Vitamin C und Bestandteile, die in Acerola-Fruchtsaft enthalten sind.

**6.** Sprühgetrocknete Zusammensetzung gemäß Anspruch 6, wobei die Zusammensetzung keinen anderen Sprühtrocknungsträger als hydrolysiertes Kollagen enthält.

**7.** Sprühgetrocknete Zusammensetzung gemäß Anspruch 5 oder 6, wobei die Zusammensetzung keinen Sprühtrocknungsträger enthält, der aus der Gruppe ausgewählt wurde, die aus Maltodextrin, Dextrin, oxidierter Stärke, Laktose, pyrogenes Siliciumdioxid, Cashewbaum-Gummi und Gummi arabicum besteht.

**8.** Sprühgetrocknete Zusammensetzung gemäß einem der Ansprüche 5 bis 7, wobei die Zusammensetzung:

(a) mindestens 0,1 Gew.-% natürliches Vitamin C umfasst;
(b) keine andere natürliche Vitamin C-Quelle als den Acerola-Fruchtextrakt umfasst; und / oder
(c) kein synthetisches Vitamin C umfasst.

**9.** Sprühgetrocknete Zusammensetzung gemäß einem der Ansprüche 5 bis 8 zur Verwendung in einem Verfahren zur Behandlung einer Krankheit oder zur Vorbeugung gegen eine Krankheit, die durch eine erhöhte Knorpelsynthese behandelt werden kann, wie Arthritis.

**10.** Nahrungsergänzungsmittel, das eine sprühgetrocknete Zusammensetzung gemäß einem der Ansprüche 5 bis 9 umfasst, wobei das Nahrungsergänzungsmittel in fester Form vorliegt, beispielsweise als Pulver, Granulat oder Tablette.

**Revendications**

**1.** Procédé pour la préparation d'une composition séchée par atomisation, comprenant un extrait de fruit d'acérola et du collagène hydrolysé, le procédé comprenant les étapes consistant à :

(a) mélanger un extrait liquide de fruit d'acérola, obtenu par pressurage d'un fruit d'acérola, et un liquide comprenant une substance issue du cartilage, comprenant du collagène de type II hydrolysé et au moins 5 % en poids du sulfate de chondroïtine liquide ;
(b) sécher par atomisation le mélange.

**2.** Procédé selon la revendication 1, dans lequel avant ou en même temps que le séchage par atomisation dans l'étape b) on n'ajoute pas de vitamine C synthétique.

**3.** Procédé selon la revendication 1 ou 2, dans lequel le liquide comprend de 20 % à 50 % en poids de collagène hydrolysé.

**4.** Procédé selon l'une quelconque des revendications 1 à 3, dans lequel on mélange l'extrait liquide de fruit d'acérola et le liquide comprenant du collagène hydrolysé en un rapport de 5:100 à 70:100 sur la base des teneurs en matière solide de l'extrait et du liquide dans l'étape a).

**5.** Composition séchée par atomisation pouvant être obtenue conformément au procédé selon l'une quelconque des revendications 1 à 4, comprenant

(a) une substance issue de cartilage, comprenant du collagène de type II hydrolysé, du sulfate de chondroïtine et
(b) de l'extrait de fruit d'acérola comprenant de la vitamine C et tous les composés différents qui sont contenus dans du jus de fruit d'acérola.

**6.** Composition séchée par atomisation selon la revendication 5, où la composition ne comprend pas de support de séchage par atomisation autre que du collagène hydrolysé ?

**7.** Composition séchée par atomisation selon la revendication 5 ou 6, où la composition ne comprend pas de support de séchage par atomisation choisi dans l'ensemble constitué par la maltodextrine, la dextrine, l'amidon oxydé, le lactose, la silice pyrogénée, la gomme de cajou, et la gomme arabique.

**8.** Composition séchée par atomisation selon l'une quelconque des revendications 5 à 7, où la composition

(a) comprend au moins 0,1 % en poids de vitamine C naturelle ;
(b) ne comprend pas de source de vitamine C naturelle autre que l'extrait de fruit d'acérola ; et/ou
(c) ne comprend pas de vitamine C synthétique.

**9.** Composition séchée par atomisation selon l'une quelconque des revendications 5 à 8, destinée à l'utilisation dans un procédé de traitement ou de prévention d'une maladie qui bénéficierait d'une synthèse accrue de cartilage, telle que l'arthrite.

**10.** Complément alimentaire comprenant une composition séchée par atomisation selon l'une quelconque des revendications 5 à 8, où le complément alimentaire est sous une forme solide, telle qu'une poudre, un granule ou un comprimé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009080778 A
- US 2003091652 A
- US 6025327 A

### Non-patent literature cited in the description

- **BENITO-RUIZ et al.** *Int J Food Sci Nutr,* 2009, vol. 60 (S2), 99-113
- **CLARK et al.** showed that collagen hydrolysate improves joint health in athletes and pain due to high athletic activity may be reduced. *Cur Med Res Op,* 2008, vol. 24 (5), 1485-1496
- **OESSER et al.** *J Nutr,* 1999, 1891-5
- **OESSER et al.** *Cell Tissue Res,* 2003, vol. 311, 393-9
- **OESSER et al.** *Orthopaedische Praxis,* 2005, 565-8
- **MOSKOWITZ.** *Semin Arthritis Rheum,* 2000, vol. 30, 87-99
- **ZUCKLEY et al.** *Med Sci Sports Exerc,* 2004, vol. 37, S 153-S4
- **NAIDU.** *Nutr J,* 2003, 7-16
- **PATEL et al.** *Ind J Sci Techn,* 2009, vol. 2 (10), 44-47
- **ASSIS et al.** *Fruits,* 2008, vol. 63 (2), 93-101
- **BOULANGER ; CROUZET.** *Food Chem,* 2001, vol. 74, 209-216
- **GENSLER et al.** *J Agric Food Chem,* 1995, vol. 43, 2662-6
- **CLARK et al.** *Curr Med Res a Opin,* 2008, vol. 24 (5), 1485-96